# EUROPEAN PATENT APPLICATION

(11) **EP 3 296 397 A1**
(43) Date of publication of application: **21.03.2018**
(21) Application number: 16189688.1
(22) Date of filing: 20.09.2016
(51) Int. Cl.: C12N 15/10

(54) **REV-CLIP, A NEW APPROACH TO IDENTIFY PROTEINS INTERACTING WITH RNA**

(71) Applicant: Danmarks Tekniske Universitet, 2800 Kgs. Lyngby (DK)
(72) Inventor: MULLER, Sébastien, 2980 Kokkedal (DK); WORKMAN, Christopher, 2800 Kgs. Lyngby (DK)
(74) Representative: Guardian IP Consulting I/S

(57) **Abstract**

The invention relates to methods for the global and specific isolation and characterization of the *in vivo* RNA-Binding Proteome (RBPome) present in a cell. The method is performed on cells that have been exposed to UV-light that serves to selectively cross-link cellular RNAs to their associated protein partners under physiological conditions. The method employs RNA splint-mediated ligation to RNA adapters, to selectively isolate cellular RNAs and their associated protein partners, and purification steps that ensure a higher yield and specificity of proteins considered as RBPs.

SEQUENCE LISTING

<110>  Danmarks Tekniske Universitet

<120>  Rev-CLIP, a new approach to identify proteins interacting with
       RNA

<130>  P2143EP00

<160>  16

<170>  PatentIn version 3.5

<210>  1
<211>  53
<212>  RNA
<213>  Artificial

<220>
<223>  Artid


<220>
<221>  misc_RNA
<222>  (1)..(53)
<223>  Artificial poly A+ RNA

<400>  1
agggcguagg cgccgugcuu uugcucggaa aaaaaaaaaa aaaaaaaaaa aaa          53


<210>  2
<211>  24
<212>  RNA
<213>  Artificial

<220>
<223>  Artificial sequence


<220>
<221>  misc_RNA
<222>  (1)..(24)
<223>  Artificial adapter sequence (RNA) modified with a 5' Phosphate
       and 3' Biotin

<400>  2
uggaauucuc gggugccaag gcau                                          24


<210>  3
<211>  49
<212>  RNA
<213>  Artificial

<220>
<223>  Artificial RNA sequence


<220>
<221>  misc_RNA
<222>  (1)..(49)
<223>  Artificial RNA Splint

<400>  3
   augccuuggc acccgagaau uccauuuuuu uuuuuuuuuu uuuuuuuuu                    49


<210>  4
<211>  20
<212>  DNA
<213>  Artificial

<220>
<223>  Artificial GAPMET RNA sequence


<220>
<221>  modified_base
<222>  (1)..(3)
<223>  cm

<220>
<221>  modified_base
<222>  (4)..(4)
<223>  um

<220>
<221>  modified_base
<222>  (5)..(5)
<223>  cm

<220>
<221>  modified_base
<222>  (17)..(17)
<223>  cm

<220>
<221>  modified_base
<222>  (18)..(18)
<223>  a=am (2'-O-methyladenosine)

<220>
<221>  modified_base
<222>  (19)..(20)
<223>  um

<400>  4
cccuctagtc ttggcucauu                                                  20


<210>  5
<211>  20
<212>  DNA
<213>  Artificial

<220>
<223>  Artificial GAPMET RNA molecule


<220>
<221>  modified_base
<222>  (1)..(1)
<223>  um

<220>

<221>  modified_base
<222>  (2)..(2)
<223>  gm

<220>
<221>  modified_base
<222>  (3)..(3)
<223>  cm

<220>
<221>  modified_base
<222>  (4)..(5)
<223>  a=am (2'-O-methyladenosine)

<220>
<221>  modified_base
<222>  (16)..(16)
<223>  cm

<220>
<221>  modified_base
<222>  (17)..(17)
<223>  a=am (2'-O-methyladenosine)

<220>
<221>  modified_base
<222>  (18)..(18)
<223>  um

<220>
<221>  modified_base
<222>  (19)..(19)
<223>  cm

<220>
<221>  modified_base
<222>  (20)..(20)
<223>  um

<400>  5
ugcaagtctg acgcccaucu                                                    20


<210>  6
<211>  20
<212>  DNA
<213>  Artificial

<220>
<223>  Artificial GAPMET RNA molecule


<220>
<221>  modified_base
<222>  (1)..(1)
<223>  um

<220>
<221>  modified_base
<222>  (2)..(3)
<223>  gm

<220>

<221>  modified_base
<222>  (4)..(4)
<223>  cm

<220>
<221>  modified_base
<222>  (5)..(5)
<223>  um

<220>
<221>  modified_base
<222>  (16)..(16)
<223>  cm

<220>
<221>  modified_base
<222>  (17)..(17)
<223>  um

<220>
<221>  modified_base
<222>  (18)..(20)
<223>  a=am(2'-O-methyladenosine)

<400>  6
uggcucaccc acgcacuaaa                                                    20


<210>  7
<211>  20
<212>  DNA
<213>  Artificial

<220>
<223>  Artificial GAPMET RNA molecule


<220>
<221>  modified_base
<222>  (1)..(1)
<223>  a=am(2'-O-methyladenosine)

<220>
<221>  modified_base
<222>  (2)..(3)
<223>  gm

<220>
<221>  modified_base
<222>  (4)..(5)
<223>  um

<220>
<221>  modified_base
<222>  (16)..(17)
<223>  a=am(2'-O-methyladenosine)

<220>
<221>  modified_base
<222>  (18)..(18)
<223>  gm

<220>

<221>  modified_base
<222>  (19)..(19)
<223>  a=am(2'-O-methyladenosine)

<220>
<221>  modified_base
<222>  (20)..(20)
<223>  cm

<400>  7
agguugcatc tggaaaagac                                                    20


<210>  8
<211>  20
<212>  DNA
<213>  Artificial

<220>
<223>  Artificial GAPMET RNA molecule


<220>
<221>  modified_base
<222>  (1)..(1)
<223>  um

<220>
<221>  modified_base
<222>  (2)..(2)
<223>  a=am(2'-O-methyladenosine)

<220>
<221>  modified_base
<222>  (3)..(4)
<223>  gm

<220>
<221>  modified_base
<222>  (5)..(5)
<223>  a=am(2'-O-methyladenosine)

<220>
<221>  modified_base
<222>  (16)..(17)
<223>  um

<220>
<221>  modified_base
<222>  (18)..(18)
<223>  gm

<220>
<221>  modified_base
<222>  (19)..(20)
<223>  um

<400>  8
uaggataata gcgctuuguu                                                    20


<210>  9
<211>  20

<212>  DNA
<213>  Artificial

<220>
<223>  Artificial GAPMET RNA molecule


<220>
<221>  modified_base
<222>  (1)..(1)
<223>  a=am(2'-O-methyladenosine)

<220>
<221>  modified_base
<222>  (2)..(2)
<223>  gm

<220>
<221>  modified_base
<222>  (3)..(5)
<223>  cm

<220>
<221>  modified_base
<222>  (16)..(16)
<223>  um

<220>
<221>  modified_base
<222>  (17)..(17)
<223>  cm

<220>
<221>  modified_base
<222>  (18)..(20)
<223>  a=am(2'-O-methyladenosine)

<400>  9
agccctctca gccacucaaa                                                    20


<210>  10
<211>  20
<212>  DNA
<213>  Artificial

<220>
<223>  Artificial GAPMET RNA molecule


<220>
<221>  modified_base
<222>  (1)..(1)
<223>  gm

<220>
<221>  modified_base
<222>  (2)..(3)
<223>  um

<220>
<221>  modified_base
<222>  (4)..(4)

<223>  cm

<220>
<221>  modified_base
<222>  (5)..(5)
<223>  a=am(2'-O-methyladenosine)

<220>
<221>  modified_base
<222>  (16)..(16)
<223>  a=am(2'-O-methyladenosine)

<220>
<221>  modified_base
<222>  (17)..(17)
<223>  um

<220>
<221>  modified_base
<222>  (18)..(19)
<223>  gm

<220>
<221>  modified_base
<222>  (20)..(20)
<223>  um

<400>  10
guucaggcat tgggaauggu                                                    20


<210>  11
<211>  20
<212>  DNA
<213>  Artificial

<220>
<223>  Artificial GAPMET RNA molecule



<220>
<221>  modified_base
<222>  (1)..(1)
<223>  cm

<220>
<221>  modified_base
<222>  (2)..(2)
<223>  um

<220>
<221>  modified_base
<222>  (3)..(3)
<223>  gm

<220>
<221>  modified_base
<222>  (4)..(4)
<223>  um

<220>
<221>  modified_base
<222>  (5)..(5)

<223>  gm

<220>
<221>  modified_base
<222>  (16)..(16)
<223>  gm

<220>
<221>  modified_base
<222>  (17)..(17)
<223>  cm

<220>
<221>  modified_base
<222>  (18)..(18)
<223>  gm

<220>
<221>  modified_base
<222>  (19)..(19)
<223>  um

<220>
<221>  modified_base
<222>  (20)..(20)
<223>  gm

<400>  11
cugugtcttg gagaggcgug                                                    20


<210>  12
<211>  20
<212>  DNA
<213>  Artificial

<220>
<223>  Artificial GAPMET RNA molecule



<220>
<221>  modified_base
<222>  (1)..(1)
<223>  a=am(2'-O-methyladenosine)

<220>
<221>  modified_base
<222>  (2)..(2)
<223>  um

<220>
<221>  modified_base
<222>  (3)..(3)
<223>  cm

<220>
<221>  modified_base
<222>  (4)..(5)
<223>  um

<220>
<221>  modified_base
<222>  (16)..(17)

<223>  gm

<220>
<221>  modified_base
<222>  (18)..(19)
<223>  a=am(2'-O-methyladenosine)

<220>
<221>  modified_base
<222>  (20)..(20)
<223>  gm

<400>  12
aucuuaatag caggaggaag                                                    20


<210>  13
<211>  20
<212>  DNA
<213>  Artificial

<220>
<223>  Artificial GAPMET RNA sequence


<220>
<221>  modified_base
<222>  (1)..(2)
<223>  gm

<220>
<221>  modified_base
<222>  (3)..(3)
<223>  a=am(2'-O-methyladenosine)

<220>
<221>  modified_base
<222>  (4)..(4)
<223>  cm

<220>
<221>  modified_base
<222>  (5)..(5)
<223>  a=am(2'-O-methyladenosine)

<220>
<221>  modified_base
<222>  (16)..(16)
<223>  cm

<220>
<221>  modified_base
<222>  (17)..(17)
<223>  a=am(2'-O-methyladenosine)

<220>
<221>  modified_base
<222>  (18)..(18)
<223>  cm

<220>
<221>  modified_base
<222>  (19)..(20)

<223>  um

<400>  13
ggacaatgac gaagccacuu                                                    20


<210>  14
<211>  20
<212>  DNA
<213>  Artificial

<220>
<223>  Artificial GAPMET RNA molecule


<220>
<221>  modified_base
<222>  (1)..(1)
<223>  a=am(2'-O-methyladenosine)

<220>
<221>  modified_base
<222>  (2)..(2)
<223>  um

<220>
<221>  modified_base
<222>  (3)..(3)
<223>  gm

<220>
<221>  modified_base
<222>  (4)..(5)
<223>  cm

<220>
<221>  modified_base
<222>  (16)..(18)
<223>  a=am(2'-O-methyladenosine)

<220>
<221>  modified_base
<222>  (19)..(20)
<223>  gm

<400>  14
augccagaaa ctgtgaaagg                                                    20


<210>  15
<211>  20
<212>  DNA
<213>  Artificial

<220>
<223>  Artificial GAPMET RNA molecule


<220>
<221>  modified_base
<222>  (1)..(2)
<223>  cm

<220>
<221>  modified_base
<222>  (3)..(4)
<223>  a=am(2′-O-methyladenosine)

<220>
<221>  modified_base
<222>  (5)..(5)
<223>  um

<220>
<221>  modified_base
<222>  (16)..(17)
<223>  cm

<220>
<221>  modified_base
<222>  (18)..(18)
<223>  a=am(2′-O-methyladenosine)

<220>
<221>  modified_base
<222>  (19)..(19)
<223>  um

<220>
<221>  modified_base
<222>  (20)..(20)
<223>  cm

<400>  15
ccaauaatcc attccccauc                                                    20


<210>  16
<211>  23
<212>  RNA
<213>  Artificial Sequence

<220>
<223>  Artificial Hammerhead ribozyme sequence


<220>
<221>  misc_RNA
<222>  (1)..(23)
<223>  Hammerhead ribozyme (RNA) region located between  5′-(Stem I)-
      and -(Stem III)-3′

<400>  16
cugaugaggc cgaaaggccg aaa                                                23

## Description

### Technical field of the invention

The invention relates to methods for the global and specific isolation and analysis of the *in vivo* RNA-Binding Proteome (RBPome) present in a cell. The method is performed on cells that have been exposed to UV-light which serves to selectively cross-link cellular RNAs to their associated protein partners under physiological conditions. The method employs RNA splint-mediated ligation to RNA adapters, to selectively isolate cellular RNAs with their associated protein partners, and purification steps that ensure a higher yield and purity of proteins representing a cell's RBPome.

### Background of the invention

The human species has undergone strong adaptive pressure leading to the establishment of complex specialized pathways, and yet the protein-coding content of the human genome does not differ greatly from that of mouse, fly, fish or worm. A mechanism that may support this higher complexity in humans is the fine-tuned regulation of these proteins rather than their raw increase in number. Alternative splicing, which allows for multiple isoforms from a single gene, and the use of a vast repertoire of non-coding RNAs (ncRNAs) represent some of the major mechanisms associated with this complexity, both of which are only superficially understood. Alternative splicing is orchestrated by precise interactions between pre-mRNA and a host of RNA-binding proteins (RBPs), while the majority of studied ncRNA species - and supposedly a large number of yet poorly explored long non coding RNAs (lincRNAs) - exert their functions by interfacing with RNA Bind Proteins (RBPs). Furthermore, RBPs are also involved in alternative poly-adenylation, mRNA localization, storage, degradation and modification; and RBPs endowed with catalytic activity may use RNA as an effector.

RBPs are essential partners for both mRNAs and ncRNAs, and the dysregulation of these interactions are implicated as the origin of certain diseases, for instance, with brain and central nervous system (CNS) diseases. Examples include TARDBP and FUS involved in amyotrophic lateral sclerosis RBFOX and FMR1 in autism and many more RBPs are expected to be directly involved. Dissecting the RNA-protein interactome network is thus a central challenge of modern biology.

RBPs specifically recognize RNAs thanks to a modular structural interface named RNA Binding Domain or RBD. Different RBD families have been characterized and have been used to identify new RBPs by homology. The combination of in silico screenings and experimental evidences suggest that human and other higher eukaryotes may display more than 1500 RBPs. The lack of efficient genome-scale methods might explain why many of the RNA-protein interactions elude characterization [1] [2] [3].

The protein-centric method of UV Cross-Linking combined with Immuno-Precipitation, CLIP, and its derived methods, has provided a basis for identifying those RNAs interacting with specific selected RNPs [4] [5] [6]. However, selecting an RBP for such a study is limited by the absence of a comprehensive RBP list and the challenges of directly identifying a connection between RBPs and disease [2]. These gaps in our knowledge could be addressed by implementing an efficient RNA-centric method capable of identifying proteins interacting with cellular RNAs [3]. Methods such as the yeast triple-hybrid, protein microarrays, and the aptamer-based capture can be used to identify candidate RBPs, but each of these methods suffer from the drawback of being low-throughput, as well as revealing non-physiological interactions, leading to many false positives [7] [8] [9] [3] [1]. A recent approach based on oligo-dT capture after UV cross-linking has extended the list of RBPs [10] [11] [12] [13] [14] [15] [16]. However, this method also, unexpectedly, detected several transcriptional factors, ribosomal proteins, enzymes involved in metabolism and non-RNA related proteins. Additionally, this method, also called RNA interactome capture, gives a global overview of the interacting partners, but cannot center on a precise transcript and its interacting RBPs. Accordingly, there exists a need for a more specific and high through-put method for detection of RBPs as well as RBPs interacting with a specific RNA transcript.

The Rev-CLIP method is principally designed to capture proteins bound to poly-adenylated RNAs (polyA+ RNAs). PolyA+ RNAs are usually assigned to the broad definition of "mRNA" but also include non-coding RNAs transcribed by RNA polymerase II, i.e. primarily long non-coding RNAs (IncRNAs). While polyA+ RNAs harbor the greatest complexity in the cell, comprising more than 80,000 different mRNAs and more than 50,000 IncRNAs, overall they represent less than 5% of total RNA, evidencing the need for very sensitive but also highly specific methods for analysis of their RBPs. However, a variant of the Rev-CLIP can target non-polyA RNAs as well.

### Summary of the invention

The invention, according to a first and second embodiment, provides A method for isolating cellular RNA Binding Proteins comprising:
a. UV cross-linking a sample of cells;
b. Preparing a lysate of the cells obtained from step (a);
c. Digesting the cell lysate obtained from step (b) with DNase;
d. Annealing target RNA molecules in the cell lysate from step (c) to RNA splint molecules, and splint ligating the annealed target RNA molecules to RNA adapter molecules to form target RNA: RNA adapter molecules; wherein the RNA adapter molecules are annealed to the RNA splint molecules before, simultaneously, or after annealing of the target RNA molecules to the RNA split oligonucleotides;
e. Immobilizing the target RNA: RNA adapter molecules of step (d) on a solid phase;
f. Washing the immobilized target RNA: RNA adapter molecules of step (e) under denaturing conditions; and either
g. Digesting the washed immobilized target RNA: RNA adapter molecules of step (f) with non-specific RNase to release RNA Binding Proteins; or,
h. Providing antisense DNA-based oligonucleotides (or GAPMET) comprising a nucleotide sequence complementary to an RNA sequence of a selected species of target RNA molecule in the washed immobilized target RNA: RNA adapter molecules of step (f); and then annealing said oligonucleotides to said target RNA:RNA adapter molecules to form complementary DNA:RNA heteroduplexes, and then digesting the DNA:RNA heteroduplexes by adding RNaseH, to release RNA Binding Proteins bound to the selected species of target RNA molecule, or,
i. Providing a trans-acting sequence-specific ribozyme comprising 5' and 3' antisense oligonucleotides, where the antisense oligonucleotides anneal to the ribonucleotides abutting the cleavage site of the selected species of target RNA molecule, to release RNA Binding Proteins bound to the selected species of target RNA molecule.

### Description of the invention

### Figures

**Figure 1****:** Cartoon showing the Rev-CLIP method
**Figure 2****:** Cartoon of the different potential splint ligations
**Figure 3** [Upper part] Cartoon showing the components of an *in vitro* test of the efficiency of the splint ligation, whereby the RNA oligo (R), an oligonucleotide mimicking a polyA+ RNA, is annealed to the RNA splint (S) and ligated to the RNA adapter (B) by a T4 RNA ligase 2. [Lower part] Image showing the ligation products separated on a denaturing urea 15% polyacrylamide gel stained by ethidium bromide. R, B, S and L were used in the indicated combinations.
**Figure 4** Image of a Western blot of SDS-PAGE-separated proteins derived from a Human Embryonic Kidney 293 (HEK) cell lysate; and HEK RBPs captured and eluted using Rev-CLIP. The image shows the detection of a negative control, β-actin, and the expected RBPs: polypyrimidine tract binding protein 1 (PTBP1) and CUG Triplet Repeat, RNA-Binding Protein 1 (CELF1).
**Figure 5** Images of a silver stained (A) and Western-Blot (B) of SDS-PAGE-separated proteins derived from HEK cell lysate, from the last wash before elution or from the eluate recovered by the Rev-CLIP capture, with or without prior exposure to UV cross-linking.
**Figure 6** Venn diagrams showing the overlap between the HEK and MIN6 RBPome obtained with Rev-CLIP, and either the 416 RNA Binding Domain (RBD)-containing proteins from human cells in the RBPDB database [17] or an ensemble of the "RNA binding" as GO terms.
**Figure 7** Histogram showing the number of proteins known as mRNA binders or non-mRNA binders in the RBPome obtained from HEK and MIN6 cells using the Rev-CLIP method as compared to proteins identified in the RBPome of HEK [10], HeLa [11], HuH7 (hepatocyte derived cellular carcinoma cell line) [14], and mouse embryonic stem cell line (mESC) [13], using the interactome capture method (IC).
**Figure 8** Histograms showing the number (A) and the ratio (B) of proteins known to be DNA binders, rRNA binders or proteins known to bind other types of non-polyA RNAs (tRNAs, snoRNAs, snRNAs, miRNAs, siRNAs) in the RBPome obtained from HEK and MIN6 cells using Rev-CLIP as compared to proteins identified in the RBPome of HEK [10], HeLa [11], HuH7 (hepatocyte derived cellular carcinoma cell line) [14], and mouse embryonic stem cell line (mESC) [13] using the interactome capture method (IC).
**Figure 9** Cartoon showing *in vitro* site-specific cleavage of a target RNA molecule using a trans-acting hammerhead ribozyme. The ribozyme oligonucleotide selectively anneals to the nucleotide sequence abutting the cleavage site in the target RNA molecule.

### Abbreviations and terms:

**Affinity group:** is a component of (or is linked to) an RNA adapter molecule; and is characterised by an affinity for a capture molecule. For example Biotin is an affinity group that has affinity for the capture molecule streptavidin and may be linked to an RNA adapter molecule.

**Capture means:** is a capture agent suitable for capture of an RNA adapter molecule comprising an affinity group, and for use in affinity purification of RNA ligated to an RNA adapter molecule.

**Protein denaturing washing conditions:** are conditions that employ a high salt wash solution comprising at least: one or more detergents, a high-salt concentration, and one or more reducing agents. An example of a high salt wash solution is a buffer comprising 50mM Tris-HCl, pH 7.4; 1M NaCl; 1mM EDTA; 0.1% SDS; 0.5% Sodium deoxycholate; and 1% nonyl phenoxypolyethoxylethanol (NP-40), 5mM DTT.

**Gapmet:** is a DNA-based oligonucleotide of 20 to 30 nucleotides in length, containing a core of 7-12 deoxynucleotides flanked at 5' and 3' ends by a sequence of 5 to 10 2'-O-methylated ribonucleotides.

**RBP:** RNA Binding Protein.

**RBPome:** ensemble of RBPs in a given cell type.

**RBD:** RNA Binding Domain.

**RNA adapter molecule:** a non-DNase degradable oligonucleotide (e.g. RNA) comprising an affinity group which can be affinity purified.

**Splint molecule:** a non-DNase degradable oligonucleotide for guiding the positioning of the RNA adapter molecule next to the target RNA molecule (e.g. a polyA+ RNA molecule), and thereby enabling RNA ligase-mediated ligation of the target RNA molecule to the RNA adapter molecule. An RNA splint molecule is an example of a suitable non-DNase degradable oligonucleotide.

**Splint ligating:** is the ligase-mediated ligation of a target molecule to an adapter molecule, wherein the respective target molecule and adapter molecule are annealed to a splint molecule.

### Detailed description of the invention

### I. The Rev-CLIP method for isolating cellular RBPs

The present invention relates to methods for the global and specific analysis of the *in vivo* RBPome present in a cell. The invention provides a new method, referred to as Rev-CLIP, to capture proteins interacting with target RNAs, in particular polyA+ RNA. The Rev-CLIP is the RNA-centric complement of the sequencing of "Cross-Linked Immune-Precipitated RNAs", known as the CLIP method [4].

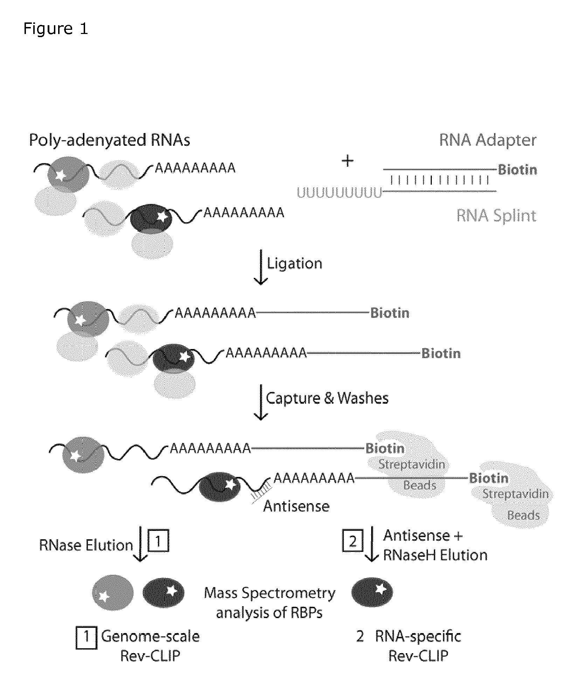

The Rev-CLIP method is performed on cells that have been exposed to UV-light, in order to selectively cross-link cellular RNAs to their associated protein partners under physiological conditions; and comprises the following steps (illustrated in Figure 1):
*Step (a) of the method:* a sample of living cells, for example a confluent cell culture, is exposed to UV light (preferably light at a wavelength of 254nm), thereby inducing the formation of covalent bounds between RNA or DNA and proteins according to the zero distance rule. Typically, UV-treatment is provided at a power not exceeding 600 mJ/cm². This UV exposure cannot lead to protein-protein cross-linking [18]. The UV-treated cells are then harvested, and optionally frozen.
*Step (b) of the method:* The harvested UV-cross-linked cells are then lysed in an EDTA-free lysis buffer comprising a protease inhibitor (for example 20mM TrisHCl pH7.5; 100mM LiCl; 0.5% w/v LiDS; 5mM DTT; EDTA-free protease inhibitor cocktail (supplied by Roche); 5mM ribonucleoside vanadyl complex (VRC).
*Step (c) of the method:* The lysate of UV-cross-linked cells is then treated with RNase-free DNase 1 (EC. 3.1.21.1) and incubated at 37°C, (preferably a DNAse 1 that is stable at high salt concentrations, such as TurboDNase supplied by Thermofisher). The DNase 1 treatment provides several important advantages to the Rev-CLIP method. Firstly, it removes DNA molecules from the lysate that might otherwise bind to the RNA splint molecule used for target RNA (e.g. polyA+ RNA) molecule capture; and thereby reduces contamination of the RBPome with DNA binding proteins. Secondly, DNase treatment significantly reduces the viscosity of the cell lysate, thereby facilitating the subsequent steps of washing the mRNA captured by Rev-CLIP.
*Step (d) of the method:* The target RNA (e.g. polyA+ RNA) molecules and associated cross-linked RBPs, present in the DNase-treated and UV-cross-linked cell lysate, are then ligated to an RNA adapter molecule by means of an RNA splint molecule and an RNA ligase. Typically, an annealing mix, comprising equimolar amounts of an RNA adapter molecule and an RNA splint molecule, is added to the cell lysate from step (c). The mixture is incubated under conditions that facilitate annealing of the target RNA molecules to a complementary sequence in the RNA splint molecules. Suitable annealing conditions include incubation in an annealing buffer (e.g. 75mM KCI; 20mM TrisHCl ph7.5), first incubating at an elevated temperature (e.g. at least 80°C), then decreasing the temperature to about 37°C, and then cooling to below 5°C, over a period of at least 10 minutes.

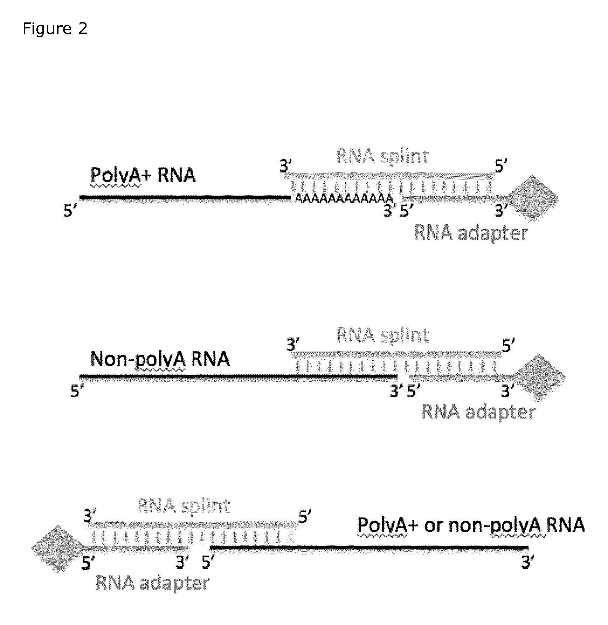

The RNA splint molecule comprises two domains that are designed to anneal to complementary sequences in the target RNA molecule and the RNA adapter molecule, such that when annealed, the base-paired terminal nucleotide of the target RNA molecule and the adjacent base-paired terminal nucleotide of the RNA adapter molecule are in sufficient proximity to permit their ligation together by means of an RNA ligase (see Figure 2).

As stated above, the RNA splint molecule comprises a domain whose nucleotide sequence is capable of annealing to the target RNA molecule in the cell lysate. An RNA splint molecule, designed to anneal with polyA+ RNA molecules comprising a 3' polyA+ tail, will typically comprise a complementary 3' terminal domain having about 20-25 uridine nucleotide residues. An RNA splint molecule comprising a 3' terminal uracil domain is particularly suitable for creating a global RBPome encompassing all RBPs in direct contact with RNA polymerase II transcribed RNAs. Alternatively, the sequence of the RNA splint molecule may be designed to anneal to a 5' terminal RNA sequence that is specific for a given target RNA molecule. Such a splint would display a 5' terminal sequence complementary to the sequence of the 5' end of the specific target RNA molecule. In an alternative embodiment, splint ligation could also be utilised to target the specific 3' end sequence of transcripts which are not poly-adenylated (for example, if transcribed by a polymerase other than RNA pol II or if the polyA tail is removed by any cellular process). Accordingly, an RNA splint molecule designed to anneal to the 3' end of a specific non-polyA RNA molecule would display a 3' end sequence complementary to the 3' end sequence of the target non-polyA RNA molecule.

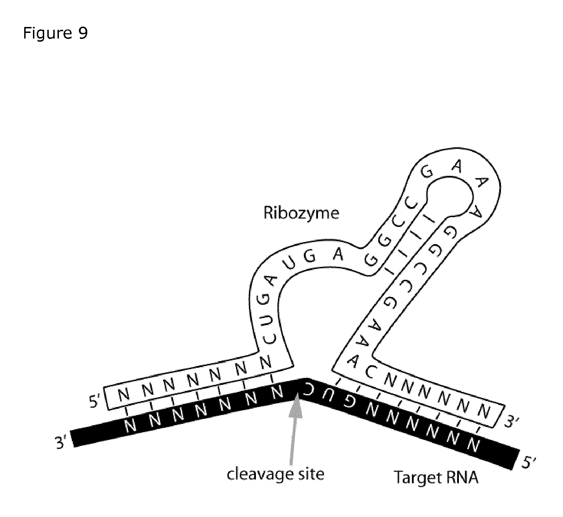

The design of a specific splint RNA molecule requires that the terminal nucleotide of the 5' or 3' end sequence of the target RNA molecule to which the splint will anneal, is known. Alternatively, a new terminal nucleotide in the target RNA molecule can be created artificially by site-specific cleavage of the target RNA, thereby creating a 5' or 3' end sequence to which a complementary splint RNA molecule will anneal. Site-specific cleavage of the target RNA may be performed *in vitro,* as shown in Figure 9, on the product of step c) of the method of the invention. Sequence-specific trans-acting hammerhead ribozymes (HHR) [19] [20] are catalytic RNA oligonucleotides that are used for this reaction. The one or more HHRs are added to the cell lysate from step c) where the one or more HHRs each anneal to their respective target RNA molecules, each forming an RNA-duplex, which is then cleaved by the HHR activity. Site-specific cleavage of the target RNA creates a new 5' terminal nucleotide in the target RNA molecule, suitable for splint-ligation to the RNA adapter.

The second domain of the RNA splint molecule is designed to anneal to the RNA adapter molecule. Typically the two domains in the RNA splint molecule will be of equal length, i.e. each about 20 - 25 nucleotides. When the RNA splint molecule is designed to anneal to the 3' end of a RNA molecule, then the sequence specifically complementary to the target RNA molecule will be the 3' end of the RNA splint. Conversely, when the RNA splint molecule is designed to anneal to the 5' end of an RNA molecule, then the sequence specifically complementary to the target RNA molecule will be the 5' end of the RNA splint. The sequence of the second domain of the RNA splint molecule is complementary to the sequence of the RNA adapter molecule. The two domains of the RNA splint molecule are contiguous, so that the RNA splint will bring the 3' OH end of the target RNA molecule and the 5' phosphate end of the RNA adapter molecule in close vicinity mandatory for ligation, or the opposite if designed to ligate the 5'end of the target RNA molecule. T4 RNA ligase 2 is then used to covalently link the 3' OH group to the 5' phosphate group without requiring any additional molecule except for ATP.

The RNA adapter molecule is designed to anneal to the RNA splint molecule, and accordingly comprises a sequence complementary to half (second domain) of the RNA splint. If the strategy is to target the 3' end or the 5' end of a given RNA molecule, the RNA adapter molecule will anneal, respectively, to the 5' or the 3' end of the RNA splint molecule. The RNA adapter molecule further comprises an affinity group. Suitable affinity groups are known in the art, but a preference is given to molecules displaying strong affinity for a capture agent, such as the affinity group biotin for the capture agent streptavidin, or RNA aptamers for specific peptides. The capture agent is generally immobilized by being attached on beads. For example, biotin can be chemically linked to one end of the RNA adapter molecule, while streptavidin is linked to beads. Use of affinity groups having a strong affinity for a capture agent ensures a complete capture of the ligated RNAs.

Once both the target RNA molecules and RNA adapter molecules are annealed to the RNA splint molecules, then the adjacent 5' and 3' terminal nucleotides of the annealed RNA adapter molecule and target RNA molecule can be ligated together using an RNA ligase (EC. 6.5.1.3.). Preferably a T4 RNA ligase2 is used, since this enzyme has a higher efficiency for ligation of double-stranded RNA templates. The RNA ligase (e.g. 10-50U T4 RNA ligase2) is added to the mixture in which the annealing reaction has taken place, and ATP is added to a final concentration about 1mM; and MgCl₂ to a final concentration about 10mM.

*Step (e) of the method:* The ligated target RNA - RNA adapter molecules (e.g. polyA+ RNA-RNA adapter molecules), obtained in step (d), are then captured by means of a capture agent. Preferably the capture agent is attached to a solid phase, such that the RNA adapter molecules can be captured on a solid phase that can be "washed" free of non-captured molecules. For example, when the capture agent (e.g. streptavidin) is itself attached to a magnetic bead, then the magnetic beads can be immobilised on a surface by a magnetic field, thereby facilitating their separation from a liquid phase either during washing or elution.

The capture agent is used to capture the ligated target RNA - RNA adapter molecules formed in the annealing/ligation reaction in the liquid medium comprising the cell lysate and an annealing buffer. The incubation conditions chosen for "capture" depend on the mode of capture and the identity of the capture agent employed. Typically "capture" is performed by adding the capture agent to the annealing/ligation liquid reaction mixture in a container and mixing the liquid overnight at 4°C. The captured ligated target RNA - RNA adapter molecules are then washed and the major part of cell lysate and the components added to mediate annealing and ligation are discarded. When the RNA adapter contains biotin and the capture agents are streptavidin magnetic beads, then the beads can be captured on the wall of the container by means of a magnet. The use of magnetic beads assists the subsequent step of washing the captured target RNA molecules free of previous reaction components. Alternatively, the washing steps can be performed by using a capture agent immobilised on a solid surface.

The washing procedure plays a key role in the specificity of the Rev-CLIP method, since it facilitates the removal of molecules that are neither target RNA (e.g. polyA+ RNA) molecules derived from the cell lysate, nor associated cross-linked RBPs that make up the cell's RBPome.

Typical sources of contamination are DNA, abundant non-polyA RNA molecules such as ribosomal (rRNA), transfer (tRNA), small nuclear (snRNA), small nucleolar (snoRNA), and micro (miRNA) RNAs and the binding proteins associated with each of these sources of contamination. While single-stranded DNA and RNA molecules might anneal to the RNA splint molecule employed in step (d), the RNA specificity of the RNA ligase will prevent its ligation to any DNA molecule annealed to the RNA adapter molecule. In the case of annealed single-stranded RNA molecules, the risk of annealing and subsequent ligation is dependent on the sequence of the RNA splint. Generally, where the RNA splint has a polyU domain for polyA+ RNA binding, the risk of binding and subsequently ligating RNA molecules lacking a polyA tail is reduced.

The washing procedure is designed, not only to remove unbound components of the cell lysate, but even more importantly to destabilise any non-ligated DNA or RNA molecules that have annealed to the RNA adapter or RNA splint molecules. Since the target RNA molecules annealed to the RNA splint molecules have also been ligated to the RNA adapter molecule, these captured RNA molecules cannot be released by the washing procedure. A suitable washing procedure comprises sequentially incubating the captured target RNA-RNA adapter molecules in a buffers having a high salt content and then a low salt content (see below), typically at low temperature; combined with exchanging the sample container with a new container. The use of high salt washes is expected to remove non-specific protein interactions, while the use of low salt washes is expected to remove the RNA-RNA or DNA-RNA interactions which occurred during the annealing step.

The essential components of wash solutions used in (e) are: i) a High-Salt wash buffer, and 2) a No-Salt wash buffer, each comprising one or more detergents, and ethylenediaminetetraacetic acid (EDTA). An example of a suitable series of washing buffers is: 1) a High Salt wash buffer (50mM Tris-HCl, pH 7.4; 1M NaCl; 1mM EDTA; 0.1% SDS; 0.5% Sodium deoxycholate; 1% nonyl phenoxypolyethoxylethanol (NP-40); 2) a No-Salt wash buffer (20mM Tris-HCl Ph7.4; 0.2% Tween 20 [2-[2-[3,4-bis(2-hydroxyethoxy)oxolan-2-yl]-2-(2-hydroxyethoxy)ethoxy]ethyl dodecanoate]); and finally 3) an RNase-compatible wash buffer (10mM Tris-HCl pH 7.4, 150mM NaCl, 0,05% NP40, 2mM EDTA).

The presence of detergents in the wash solution ensures denaturing conditions for all proteins. The role of these detergents is three-fold. 1) Detergent completely unfolds the proteins and thereby prevents the binding of proteins to any available RNAs during the course of the washes. Such non-physiological interactions are common artefacts observed in protocols in which stringent washes cannot be applied. Since the capture is based on covalent bonds between RBPs and the target RNA molecules, and between target RNA molecules and the adapter RNA molecules, and a very strong binding between the affinity group (e.g. biotin) present on the adapter RNA molecule and the capture agent (e.g. streptavidin beads), extremely harsh washes can be used to avoid these artefacts. 2) Detergent prevents protein-protein interactions so that proteins recovered after elution are *bona fide* RBPs cross-linked to the target RNA or polyA+ RNA molecules rather than proteins with affinity for any recovered RBPs. Additionally, the presence of reducing agents (e.g. dithiothreitol, DTT) helps to dissociate any tertiary structure of the proteins and thereby decreases the risk of non-RBP proteins forming pockets capable of retaining the target RNA molecules or RNA adapter molecules. 3) Detergents prevent non-specific binding of proteins to the walls of reaction vessels or to capture agent beads. The washing solution components not only contribute to increasing the specificity of the Rev-CLIP method, but also to countering the loss of RBPs during the course of the purification.

During the process of the purification, the protection of target RNA molecules is essential. Protection is initially provided by ribonucleoside vanadyl complex (VRC) present in the lysis buffer. It is crucial to consider two points regarding VRC. Firstly, VRC dissociates from RNA in presence of excess of EDTA. EDTA is also used to chelate divalent ions to protect RNAs when exposed to temperature above 45°C. Secondly, VRC will inhibit non-specific RNAses used for subsequent elution steps, while the activity of RNase H is not affected. As a consequence, the presence of VRC must be controlled carefully. VRC is initially added with the lysis buffer to counter the endogenous RNAses but is progressively removed by the successive washes containing EDTA, which also serve to progressively remove endogenous RNases. It is therefore important to maintain an RNase-free environment throughout the REV-CLIP method, since the target RNA molecules lose this protection during the course of the purification, in order to be degraded by the exogenous RNases added in the final elution step.

Furthermore, in order to eliminate potential DNA binding proteins, DNase treatments can be extensively used in step c) since no DNA *per se* is used for the capture of the target RNA molecules. However, the subsequent washes in step (e) are important so that no DNase enzyme remains that might otherwise interfere with an RNaseH-based elution.

*Step (f) of the method:* The target RNA molecules and their associated RBPs captured from the cell lysate in step (e), can either be treated to release all cellular RBPs referred to as RBPome (according to a first embodiment); or specific species of captured target RNA molecules and their associated RBPs can be released (according to a second embodiment).

Thus, according to the first embodiment, the captured target RNA-RNA adapter molecules are digested with a non-specific RNase, for example RNaseI (EC. 3.1.27.5) or RNaseT1 (EC: 3.1.27.3) at about 37°C, which releases the RBPs associated with all the captured target RNA-RNA adapter molecules. The RBPs can then be concentrated, for instance, by acetone precipitation.

Thus, according to the second embodiment, a selected species of target RNA molecule is released by means of an exogenous RNaseH (EC. 3.1.26.4) guided by the presence of an antisense oligonucleotide. Several antisense oligonucleotides can be used sequentially to target different regions of the same target RNA molecule or additional species of target RNA molecule, thereby accelerating the throughput of the characterization of these target RNA molecules. The present invention provides a new type of antisense oligonucleotide, referred to as gapmet. Gapmets are 20 - 30nt oligonucleotides in length (e.g. 20 nt) harboring a 7-12 nt DNA core (e.g. 10nt), flanked at 5' and 3' ends by 5-10 (e.g. 5) 2'-O-methylated RNA residues. By limiting the potential DNA-RNA hetero-duplex to 12nt (preferably 10nt), the gapmets tend to increase the specificity by guiding RNaseH activity only when most, if not all, of its nucleotide residues are base-paired to target RNA molecules displaying complementary sequences. Indeed, a weaker and non-specific base-pairing, for instance of only the 10 5'nucleotides of the gapmet, would not create a sufficient RNA-DNA duplex for the action of RNaseH (which may need to recognize at least 7-8 DNA-RNA paired-bases), although oligonucleotides made only of unmodified DNA would be sufficient. Once a given species of target RNA molecule has been released together with its associated RBPs, then the target RNA molecule is degraded using non-specific RNases (e.g.RNaseI/RNaseT1) and the RBPs can be concentrated.

The specific release of a selected species RNA target molecule, according to the second embodiment, may alternatively be performed by providing one or more trans-acting sequence-specific ribozyme (e.g. hammerhead ribozyme) comprising 5' and 3' antisense oligonucleotides, where the antisense oligonucleotides anneal to the ribonucleotides abutting the cleavage site of the selected species of target RNA molecule, to release RNA Binding Proteins bound to the selected species of target RNA molecule.

In order to confirm the release of the captured target RNA molecules, a fraction of the captured RNA molecules can be treated so that intact or mildly digested target RNA molecules are released and further analysed by RNA-sequencing or RT-PCR. The RNA elution can be performed by proteolytically digesting the affinity group-capture agent (e.g. biotin and streptavidin) as well as the associated RBPs by proteinase K treatment (EC. 3.4.21.62). A mild RNA digestion using low amounts of non-specific RNase can favour the RNA elution since the streptavidin core interacting with biotin can be difficult to degrade proteolytically. Then, the released target RNA molecules can be selectively extracted by phenol/chloroform separation and recovered by ethanol precipitation.

*Analysis of the global RBPome:* The RBPs obtained according to the first embodiment of step (f) represents the cell's genome-scale RBPome, which can be analysed and identified, for example by Mass Spectrometry.

*Analysis of the mRNA species-specific RBPome:* The RBPs obtained according to the second embodiment of step (f) represent the group of RBPs playing a role in the processing, expression or function of specific species of target RNA molecule (e.g. polyA+ RNA), which can also be analysed and identified, for example by Mass Spectrometry.

The Rev-CLIP method has been applied to isolate the RBPome of HEK and MIN6 cells, and the composition of the isolated RBPomes has been determined and compared with the RBPome isolated using methods in the prior art. The Rev-CLIP method is shown to provide a surprising improvement over the IC method, particularly in respect of the selectivity for RBPs associated with polyA+ RNA molecules; and the low level of contamination with proteins known to bind to DNA and non-polyA or non-targeted RNA molecules.

Furthermore, the RevCLIP method is shown to be sufficiently sensitive to be able to detect slight changes in RBP content in mouse ß cells (MIN6) in response to exposure to low or high glucose concentrations. Indeed, Rev-CLIP allows the identification of RBPs directly involved in the response to glucose uptake, thereby identifying protein targets relevant for treatment of type II diabetes.

Finally the RNA-specific Rev-CLIP has been performed on HEK cells by targeting 4 candidates lincRNAs in order to characterise the direct RBP protein partners.

### II. Cells for analysis of RBPome using Rev CLIP

The Rev-CLIP method can be used to isolate the RBPome of all cells that can be cultured *ex-vivo* and *in vitro,* including cells of eukaryotic and prokaryotic origin. For example, the Rev-CLIP method can be applied to bacterial, mycobacterial and yeast cells. Since an analysis of the RBPome can be used to identify future therapeutic targets, the Rev-CLIP method is advantageously applied to cells of mammalian origin. In particular, Rev-CLIP method may be applied to any cell type, for example, of mouse, rat or human origin.

Suitable methods for cultivation of cells of mammalian origin are well known in the art.

### Examples

### Example 1 Construction and in vitro testing of a biotinylated RNA adapter and splint for capturing polyA+ RNA

A biotinylated RNA adapter in combination with a splint RNA were synthesized and tested for use in the selective capture of polyadenylated RNA. The RNA adapter and splint were tested using an artificially poly-adenylated RNA as a mimic for polyA+ RNAs present in a cell lysate. The artificial polyA+ RNA comprised a 53nt long RNA oligonucleotide harboring a 3' tail of 25 Adenine residues, having the sequence:
5'-AGGGCGUAGGCGCCGUGCUUUUGCUCGG(A)₂₅-3' [SEQ ID No: 1]

The sequence of the RNA adapter and RNA splint are as follows:
Adapter sequence (RNA): 5' Phosphate-UGGAAUUCUCGGGUGCCAAGGCAU-Biotin-3' [SEQ ID No: 2]

### Splint sequence (RNA): 5'-AUGCCUUGGCACCCGAGAAUUCCA(U)₂₅-3' [SEQ ID No: 3]

Hammerhead ribozyme (RNA): 5'-(Stem I)-CUGAUGAGGCCGAAAGGCCGAAA-(Stem III)-3' [SEQ ID No:16] where Stem I is 4-10 nucleotides complementary to the sequence of the target RNA molecule that is 3' of the cleavage site and Stem III, starting with C or U, that contains 4-10 additional nucleotides complementary to the target RNA molecule that is 5' of the cleavage site C, where the cleavage site is unbasepaired and lies between Stem I and Stem III (see Figure 9).

PolyA+ RNA capture and splint-ligation were tested by preparing a series of annealing mixes, where one or more components required for polyA+ RNA capture and ligation were omitted. An annealing reaction was performed to anneal the RNA adapter to the RNA splint as follows: 50pmol of the biotinylated RNA adapter was combined with 50pmol of the splint RNA in 30µl total annealing buffer (75mM KCI; 20mM TrisHCl pH7.5). This first annealing reaction mix was incubated for 1min at 85°C, then for 3min at 65°C, 10min at 37°C, and finally on ice for 5min. The RNA adapter-splint was then annealed to the artificial polyA+ RNA by combining 30µl of the first annealing reaction with 50pmol artificial polyA+ RNA (suspended in 40µl) in a 1.5ml tube and incubating this second annealing reaction mixture for 3 min at 55°C, then 10 min at 37°C, and finally 10 min on ice. The products of the second annealing reaction were then ligated by adding 30U of T4RNA ligase2 (supplied by MClab) in a ligation reaction comprising 1mM ATP, 10mM MgCl2, followed by incubation at 37°C for 2h.

The ligation reaction products were then denatured for 2min at 90°C in formamide blue (30% deionized formamide; 6mM EDTA; bromophenol blue) and analysed by separation on a denaturing 15% urea PAGE pre-warmed at 50°C.

### Results:

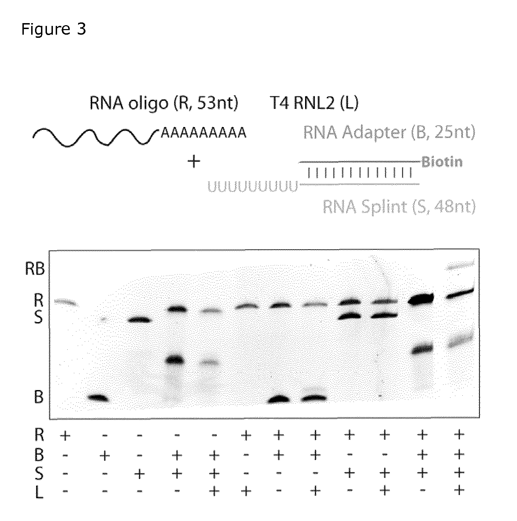

As seen in Figure 3 (bottom part), a ligation reaction product (RB) whose migration corresponds to the expected size of polyA+ RNA (R) ligated to the RNA adapter was detected following PAGE separation under denaturing conditions. This ligated product was only obtained when the polyA+ RNA (R) annealing steps were performed in the presence of both the RNA adapter (B) and the RNA splint (S), and the ligation step was performed in the presence of T4 RNA ligase2. Furthermore, the T4 RNA ligase2 could not ligate the RNA adapter to the single stranded polyA+ RNA, but instead required the presence of the RNA splint.

### Example 2 The Rev-CLIP method was tested and shown to capture polyA+ RNA-bound RBPs in cell lysates.

The Rev-CLIP method was applied to Human Embryonic Kidney (HEK) cells and tested for its ability to capture specific polyA+ RNA-bound RBPs known to be present in the RBPome of HEK cells.

*HEK cell culture:* A culture of HEK cells was obtained by culturing HEK cells in pyruvate buffered Dulbecco's Modified Eagle's Medium (DMEM) supplemented with 10% heat inactivated FBS (supplied by Gibco), Penicillin-Streptomycin and L-glutamin, in Nunclon Delta-treated flasks (supplied by Thermoscientific) or TC treated dishes (supplied by Corning).

The following method steps were followed:
*Step (a): UV cross-linking of RBPome of HEK cells.* Prior to UV cross-linking, cells were grown in 500cm² plates (2x10⁷ cells) until 90% confluency. The confluent cells were then washed in PBS, and then exposed to 150mJ/cm² UV light at 254nm, 4 times, using a UV box (UVP CL-1000). The cells were then immediately scraped off the plates to avoid any degradation of RNAs and proteins; collected into 50ml Falcon tubes; and centrifuged at 1500g, for 10 min at 4°C (HEK). The cell pellets, so obtained, were then frozen at -80°C until use.

The Rev-CLIP method was applied to the UV-cross linked cells as followed:
*Step (b): Frozen lysates prepared from UV-crosslinked cells.* Frozen cells, derived from 3x10⁸ cells (corresponding to confluent cell growth on 15 plates of 500cm²) were re-suspended in Lysis buffer (20mM TrisHCl pH7.5; 100mM LiCl; 0.5% w/v LiDS; 5mM DTT; protease inhibitor; 5mM ribonucleoside vanadyl complex (VRC)); transferred to a Potter-Elvehjem glass tube; and dounced 20 times with a PTFE pestle. In general, 5ml of Lysis buffer were sufficient per pellet (corresponding to 2x10⁷ cells from one 500cm² plate). Lysates were then transferred to 50ml falcon tubes and incubated 10min on ice.
*Step (c): DNase treatment of UV-cross-linked cells.* The lysates were DNase treated by the addition of 5µl of turbo DNase (supplied by LifeTech) per ml of lysate and incubation for 10 min at 37°C.
*Step (d): Annealing and ligation of RNA adapter to polyA*+ *RNA in DNase-treated, UV-cross-linked HEK cell lysate.* The subsequent quantities in the method are relative to 2x10⁷ cells. An annealing mix comprising 300pmol of biotinylated RNA adapter and 300pmol of splint RNA in 30µl total annealing buffer (75mM KCI; 20mM Tris-HCl, pH7.5) was prepared; and then sequentially incubated for 1 min at 85°C; 3 min at 65°C; 10 min at 37°C, and finally 5 min on ice. Two volumes (10ml) of Buffer L (65mM Tris-HCl pH7.5; 5mM DTT; protease inhibitor) were added to 5ml of lysate.

The splint ligation was performed in two steps:
First, 30µl of annealing mix were added to 15ml of diluted lysate in a 50ml tube and then sequentially incubated for 3 min at 55°C; 10 min at 37°C; and finally 10 min on ice.
Second, 1mM ATP, 10mM MgCl2 and 30U of T4RNA ligase2 (MClab) were added to the annealed mixture, which was then incubated at 37°C for 2 h.

*Step (e): Capture of biotinylated RNA adapter.* The biotinylated RNA adapter and all polyA+ RNAs ligated to the adapter were then captured on streptavidin beads, previously equilibrated in 3 volumes of Lysis buffer, as follows. 40µl of streptavidin magnetic beads (supplied by Pierce, 88817) was added to 15ml ligated lysate obtained from step d. The capture was performed overnight at 4°C with gentle wheel rotation (7rpm). The 50ml tubes were then placed on a magnetic stand at 4°C until the beads were completely immobilized. The beads were re-suspended in lysis buffer, transferred to 1.5 ml tubes and reimmobilized using a dedicated magnet stand and the supernatant was discarded. The beads were washed in lysis buffer 3 more times for 5 min at 4°C with wheel rotation. The beads were then sequentially washed: 4 times with ice-cold High Salt buffer (50mM Tris-HCl, pH 7.4; 1M NaCl; 1mM EDTA; 0.1% SDS; 0.5% Sodium deoxycholate; 1% NP-40; 5mM DTT); twice with ice-cold No-Salt Buffer (20mM Tris-HCl Ph7.4; 0.2% Tween); and finally twice with RNase-compatible buffer (10mM Tris-HCl pH 7.4, 150mM NaCl, 0,05% NP40, 2mM EDTA). Tubes were changed between washes and protein lobinding Microcentrifuge tubes were used for the elution.

For the purpose of identifying the RBPome captured by the Rev-CLIP method, the bound RBPs were then released as follows:
*Step (f): Genome scale-elution of RBPome captured by the Rev-CLIP.* A volume of 394µl of RNase buffer supplemented by non-specific RNases: 400U of RNaseI (supplied by LifeTech) and 2000U of RNaseT1 (supplied by LifeTech) was added to the washed beads obtained in step (e). The mixture was mixed in a thermoshaker at 800rpm for 1h at 37°C. After removal of the beads using the magnetic stand, the bead-free eluate was collected in a 5ml Microcentrifuge tube; and the captured beads are again exposed to elution buffer (100µl RNase buffer), by continuous 800rpm shaking at 37°C for 20min. The first and second eluates were combined to give a final eluate volume of 500µl. The proteins in the eluate were then concentrated by adding 5 volumes of -20°C cold acetone, vortexed 5sec and incubated overnight at-20°C. Proteins were centrifuged for 15min at 15,000g and 4°C. The acetone was then removed from the protein pellet by drying for 30min at room temperature; where after the dry proteins were stored at -20°C.

### The presence of some known RBPs in the RBPome obtained from HEK cell using Rev-CLIP was tested by Western blotting

*Western blotting:* Samples, obtained in step (c) and (f) of Rev-CLIP method, were denatured in SDS loading buffer (63mM Tris HCl pH6.8; 1% SDS; 5% glycerol; 5% ß-mercaptoethanol; bromophenol blue) by shaking at 800rpm for 10min at 70°C, and then loaded on NuPAGE 4-12% gradient gels (supplied by Invitrogen), and then separated in parallel with a SeeBlue Plus2 Prestained Protein Standard (supplied by Novex). The resolved proteins were then transferred to a PVDF membrane, 0.45µm (supplied by Thermoscientific), in the presence of methanol-free transfer buffer (supplied by Pierce). Membranes are re-blotted using stripping buffer (supplied by Pierce). Transferred proteins were screened with the following antibodies: anti-ßactin (1:10,000, mouse, A2228, Sigma), anti-CELF1 (1:5,000, rabbit, ab129115, Abcam), anti-PTBP1 (1:10,000, rabbit, ab133734, Abcam), HRP-anti-mouse IgG (1:20,000, 115-035-003, supplied by Jackson Immuno Europe), HRP-anti-rabbit IgG (1:10,000, 111-035-008, supplied by Jackson Immuno Europe) using the standard Western blot protocol. Horse Radish Peroxidase (HRP) signal was revealed by use of SuperSignal West Femto Maximum Sensitivity kit (supplied by Pierce) on a G:box chemi XR5. The Silver Staining was carried out on a duplicate NuPAGE 4-12% gradient gel loaded with the same samples using Sigma Silver Staining Kit.

### MS analysis: as described for example 3.

### Results:

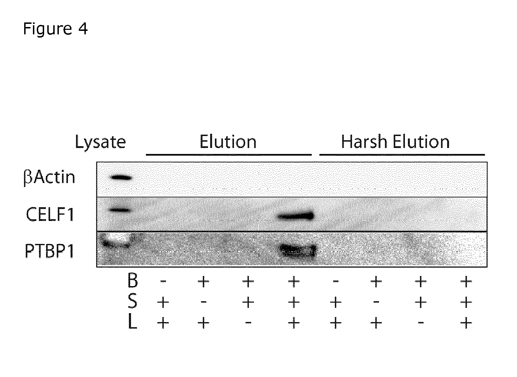

As seen in the Western blot of proteins derived from HEK cells shown in Figure 4, each of the proteins β-actin, polypyrimidine tract binding protein 1 (PTBP1) and CUG Triplet Repeat, RNA-Binding Protein 1 (CELF1) were detected in lysates of HEK cells. The two RBPs, PTBP1 and CELF1, present in the cell lysate were also selectively detected amongst the proteins captured by the Rev-CLIP method, which as expected did not include β-actin. The capture of the two RBPs by the Rev-CLIP method, was dependent on employing the RNA adapter, the RNA splint and the T4 ligase2, and omission of any one of these components prevented the capture of these RBPs. A harsh elution was additionally performed after the elution in step f), by exposing all the remaining beads to 30µl SDS loading buffer and by shaking at 800rpm for 30min at 70°C. This harsh elution entirely degrades streptavidin and therefore shows what was captured but not properly eluted from the beads by the previous step f), as well as what remained attached to the beads, mediated by RNA-independent non-specific interaction with the beads. The harsh elution revealed that the elution step was sufficiently efficient, since no more of the RBPs CELF1 or PTBP1 were observed in the harsh elution lanes (Figure 4). Overall, the experimental results demonstrate that the capture of polyA+ RNA and its associated RBPs by the Rev-CLIP method is dependent on both the annealing and splint-ligation of the polyA+ tail of the RNA molecules in the cell lysate to the RNA adapter. As a consequence, molecules of rRNA, tRNA, snRNA, snoRNA and miRNA, despite their abundance in the cell lysate, should not be captured by the RNA splint-ligation used for polyA+ capture as they lack the required long stretch of A residues at their 3' end. Since the cell lysate, used in the Rev-CLIP method, is subjected to extensive DNase treatment, the risk of recovery of DNA fragments and their associated proteins binding are drastically reduced.

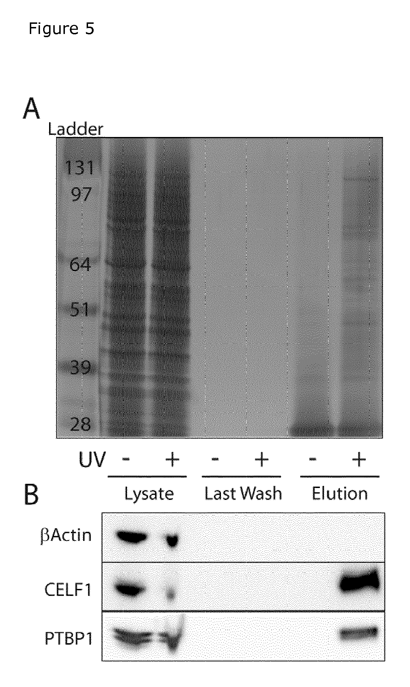

The Rev-CLIP method is performed on UV-exposed cells. The effect of this UV exposure on the results of Rev-CLIP method was analysed by the comparison of samples prepared under two conditions, UV or non-UV exposed cells. UV-cross-linking of cells in step (a) of Rev-CLIP, is performed to covalently bind RBPs to RNA under physiological conditions. The Rev-CLIP method uses harsh washes (detergents, reducing agents and high salt buffer) that denature all the proteins, and under these conditions, RBPs would lose their ability to bind to polyA+ RNAs during the purification. Only those RBPs which have been previously cross-linked to polyA+ RNA can thus be captured. After elution, the non-UV exposed cells are used as a negative control, where RBPs should not be retained due to the denaturing conditions and the stringent washes. In the case of non-UV exposure conditions, the recovered proteins are expected to be proteins that are very abundant, or to be proteins that retain the ability to stick to RNA when denatured, or simply proteins with an affinity for the beads, biotin, streptavidin and/or the plastic of the microcentrifuge tubes. For instance, proteins which may employ biotin as a cofactor might be recovered for that reason (only ten proteins are known to have such properties in humans). Overall, as visualized on a silver-stained SDS-PAGE, the proteins recovered from UV-exposed cell samples outnumber those recovered from non-UV exposed cell samples (Figure 5A). The stained gel in Figure 5A clearly shows a greater amount of protein bands released in the RNase elution buffer than from the UV-cross-linked samples. The Western blot analysis (Figure 5B) confirms the presence of RBPs, such as the two RBPs, PTBP1 and CELF1, in the lane comprising the UV-exposed sample, but not in the lane comprising the non-UV exposed sample. The Silver Staining and the Western Blot methods have a detection threshold that is much higher than that of Mass Spectrometry (MS). Nonetheless, as demonstrated in Example 3, MS detects about five times more RBP proteins when Rev-CLIP is performed on UV-exposed cell samples versus non-UV exposed samples.

### Example 3 Characterisation and quantification of RBPome detected by Rev-CLIP

The RBPome was isolated from both HEK cells, as well as mouse insulinoma-derived β-cell line26 cells (MIN6), using the Rev-CLIP method, as described in Example 2. The MIN6 cells were grown in DMEM containing 50µM ß-mercaptoethanol. The glucose concentration of the medium was adjusted to 3mM (Low), 25mM (High) or was 22mM if not mentioned. The proteome and component RBPs isolated using Rev-CLIP (samples obtained in step (f) of Rev-CLIP) were characterised and quantitated using the methods set out below.

*Sample preparation for MS:* Protein pellets (10µg) were dissolved in 22µl buffer D (8M Urea; 50mM Tris-HCl pH 8.0; 45mM DTT) and incubated 45min at room temperature. Then 4µl freshly prepared 500mM IodoAcetic Acid (IAA) were added before incubation for 1h at room temperature in the dark. Trypsin digestion was performed by addition of 74µl of 50mM Tris-HCl pH 8.0 and 2µl 0.1µg/µl trypsin dissolved in 10mM HCl, in order to get a protein/trypsin ratio of 50:1, and incubated overnight at 25°C. The digestion was stopped by addition of 2µl of 10% trifluoroacetic acid (TFA). The protein sample was concentrated using the speedvac and acidified with 2µl 10% TFA to reach pH<2. A C18 stage tipping protocol was applied. C18 filters were placed carefully at the very end of a DL-10 Diamond tower pack 10 µl tips using a sampling tool syringe. C18 filters were activated by rinsing them sequentially with wetting/elution solution (60% acetonitrile, 0.1% TFA) and equilibration solution (0.1% TFA) removed by short spin at max 4,000rpm. 5µg of peptides were rapidly loaded with a 20sec spin at 3,000rpm. The filters were washed twice with 20µl equilibration solution and stored at 4°C in a 50ml Falcon tube with 50µl equilibration solution until MS is performed. The peptide samples were eluted slowly with 10µl wetting/elution solution, using a syringe, and kept only shortly at 4°C before MS. The 10µl were almost completely dried using the speedvac. Then 2.5µl of 2% formic acid were added and the volume adjusted to 10µl with water. A sample of 0.5µl was used for Maldi analysis and the remainder for LC-MS.

*LC-MS*/*MS analysis:* Samples, prepared as above, were analyzed by liquid chromatography tandem mass spectrometry (LC-MS/MS) and data were recorded in a data dependent manner, automatically switching between MS and MS/MS acquisition, on a quadrupole mass filter instrument with an Orbitrap mass analyzer (Q-Exactive, Thermo scientific, Bremen, Germany). The liquid chromatography system was coupled to the mass spectrometer through an EASY spray source operated at 2.1kV with no sheath or auxiliary gas applied a heated capillary temperature of 275°C and an S-lens RF at 50. Liquid chromatography was performed on an EASY nLC-1000 (Thermo scientific, Odense, Denmark). Commercial 50cm EASY-spray columns (Thermo scientific ES803) were used to online separate the peptides prior to analysis. These columns have an inner diameter of 75µm and are packed with reverse phase C18 material with 2µm size particles. During elution an in build column oven kept the temperature of the column at 45°C.

The mobile phase consisted of Solvent A containing 0.1% formic acid and the peptides were eluted by an increasing gradient of Solvent B containing 80% acetonitrile in 0.1% formic acid. The total gradient was 130min and all changes of solvent mixtures were done in a linear fashion. The Solvent B concentration at the start of the gradient was 6% hereafter the gradient was as follows: 14%B in 37min, 25%B in 42min, 38%B in 21min, 60%B in 20min, 95%B in 3min and 95%B for 7min.

The full scans were acquired in the Orbitrap with a resolution of 70,000 and a predictive automatic gain control (AGC) of 3,000,000 based on the AGC from the previous full scan. If this was not reached, a maximum injection time of 20ms was applied. For the full scans the scan range was adjusted to 300-1750 m/z.

The top 10 most abundant ions from the full scan were further sequentially selected for fragmentation and MS/MS analysis with an isolation window of 1.6 m/z and a minimum intensity threshold of 100,000. Ions with unassigned charge states, charge states of 1 and isotopes of ions were excluded from selection for MS/MS analysis. Further analyzed ions were excluded from reselection for a 30 sec time period. For the MS/MS scans the resolution was adjusted to 35,000 with a predictive AGC of 1,000,000, a maximum injection time of 60ms and a fixed first mass of 120m/z. The ions were fragmented in a higher-energy collision dissociation (HCD) cell with normalized collision energy (NCE) of 25%.

*Protein identification and quantification:* MaxQuant (v1.5.2.12) was used to analyze the data. Peptides were compared against the ENSEMBL release81 GRCh38 human and Swissprot mouse proteome database for the HEK and MIN6 cells, respectively. Default parameters were used: FDR of 0.01, peptides with a minimum length of seven amino acids, cysteine carbamidomethylation set as a fixed modification, N-terminal acetylation and methionine oxidation set as variable modifications. Label-Free-Quantification (LFQ) is used. Only peptides uniquely mapping to one protein group were used for further analysis. Quantification was performed when at least one unique peptide was detected. Ion counts from three technical replicates were pooled and pseudocounts were used to calculate ratios between experiments including or no UV-crosslinking.

### Results

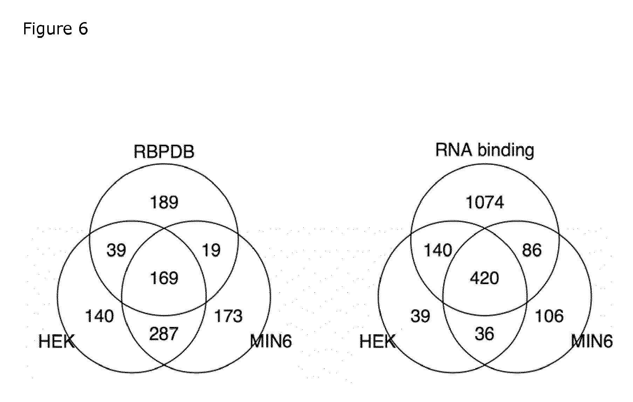

The Rev-CLIP performed in HEK and MIN6 detected, respectively, a total of 639 and 648 proteins which passed the selected cutoffs set for peptide counts (at least 2 peptides detected in the 3 biological replicates) and the fold change between UV versus non-UV exposed samples (the averaged peptide counts log2FC must be above 0.5 and the averaged LFQ intensities FC must be above 2). About 40% and 37% of the proteins identified in HEK and MIN6 RBPomes respectively, were proteins for which direct binding to polyA+ RNAs has been demonstrated experimentally in the literature. The HEK and MIN6 RBPomes identified herein show a large overlap with a database of RBD-containing proteins (RBPDB, [17]) and "RNA binding" as GO terms (figure 6).

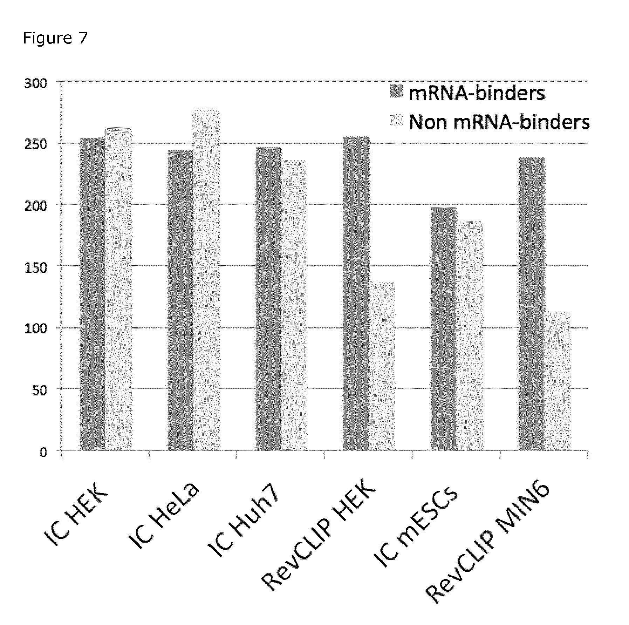

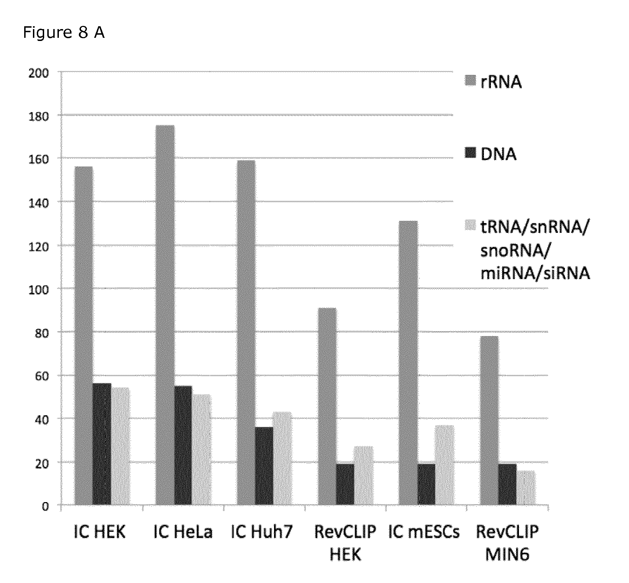

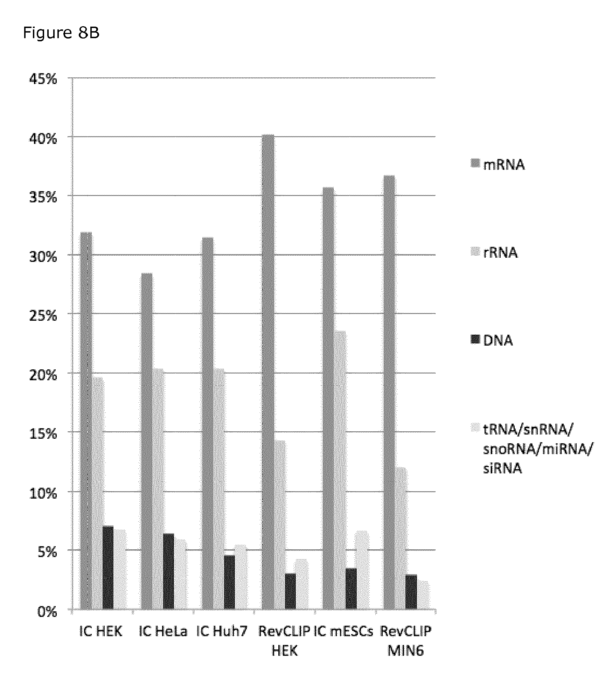

The efficacy of Rev-CLIP for specifically isolating RBPs capable of binding to mRNA was compared to methods known in the art (RNA interactome capture technique (IC)). As shown in Figure 7, Rev-CLIP facilitates the isolation of a higher proportion of RBPs capable of binding to polyA+ RNA than the IC method used for analysis of the RBPome of comparable HEK cells and other cell lines, reported in the literature. Additionally, as shown in Figure 8, Rev-CLIP enables the isolation of a population of proteins that bind to polyA+ RNA, comprising fewer proteins known to bind to non-polyA RNA (e.g.rRNA, tRNA, snoRNA, snRNA and miRNA) or DNA, as compared to proteins isolated by the IC method.

### Example 4 Specific Rev-CLIP method adapted for the isolation of RBPs bound to a specific polyA+ RNA molecule

The RBPs bound to a specific polyA+ RNA molecule can be selectively released using the specific Rev-CLIP method. According to this alternative method, all polyA+ RNAs are captured as previously described, but the elution of RBPs is limited to those bound to a given polyA+ RNA molecule.

The specific Rev-CLIP method shares the same steps (a)-(e) with the Rev-CLIP set out in Example 2. The method only diverges in step (f), at the stage of washing the beads, which was performed in buffer R' (50mM TrisHCl, pH7.5; 100mM NaCl; 3mM MgCl2). A first wash (and controlled elution) of the beads was performed by addition of 550µl Buffer R'; with mild shaking on wheel at 4°C for 5min, followed by addition of 20U RNaseH (MCLab) (8 µl). The tubes were incubated further on a wheel at 45°C for 15min, the beads were captured on a magnetic stand, and the eluate fraction collected in a tube named "elution control". The beads were then washed successively - twice for 5min at 45°C on wheel with No-Salt buffer; and then one time for 5min at 4°C; one time with High Salt buffer, and finally one time with buffer R'. The tubes were changed between washes.

The specific elution was then carried out as follows: The beads were re-suspended in 400µl buffer R' and put on wheel at 45°C for 5min. Then, 30pmol of a given RNA-DNA hybrid oligonucleotide ("gapmet", see above) was added to the re-suspended beads, and the tubes were placed on wheel at 45°C for 5min. Then 20U RNaseH were added and the tubes were incubated on the wheel at 45°C for 15min. The specific elution fractions were finally collected. A supplementary elution was performed by adding 150µl of Buffer R' on beads, incubated again at 45°C for 5min and collected into the first collected fraction. Additional specific elutions were obtained by repeating the sequence of washes and elutions with a different gapmet. For each elution fraction, the RNAs were digested in presence of 200U of RNase I plus 1000U of RNase T1 at 37°C for 20min and precipitated with 5 volumes of cold acetone. One part of the fraction (usually one tenth) was kept for RNA analysis after digestion of proteins with proteinase K as previously described.

The specific Rev-CLIP method was applied to 4 RNA species: NEAT1, HOTAIR, XIST and MALAT1, employing 3 different gapmets for each of these 4 RNAs. The sequences of the gapmets were:
Neat1-3163: 5'-CmCmCmUmCmTAGTCTTGGCUmCmAmUmUm [SEQ ID No: 4]
Neat2-2649: 5'-UmGmCmAmAmGTCTGACGCCCmAmUmCmUm [SEQ ID No: 5]
Neat3-2275: 5'-UmGmGmCmUmCACCCACGCACmUmAmAmAm [SEQ ID No: 6]
Malat1-6886: 5'-AmGmGmUmUmGCATCTGGAAAmAmGmAmCm [SEQ ID No: 7]
Malat2-7498: 5'-UmAmGmGmAmTAATAGCGCTUmUmGmUmUm [SEQ ID No: 8]
Malat3-8129: 5'-AmGmCmCmCmTCTCAGCCACUmCmAmAmAm [SEQ ID No: 9]
Hotair1-1958: 5'-GmUmUmCmAmGGCATTGGGAAmUmGmGmUm [SEQ ID No: 10]
Hotair2-1617: 5'-CmUmGmUmGmTCTTGGAGAGGmCmGmUmGm [SEQ ID No: 11]
Hotair3-1979: 5'-AmUmCmUmUmAATAGCAGGAGmGmAmAmGm [SEQ ID No: 12]
Xist-1-19024: 5'-GmGmAmCmAmATGACGAAGCCmAmCmUmUm [SEQ ID No: 13]
Xist-2-18895: 5'-AmUmGmCmCmAGAAACTGTGAmAmAmGmGm [SEQ ID No: 14]
Xist-3-18840: 5'-CmCmAmAmUmAATCCATTCCCmCmAmUmCm [SEQ ID No: 15];
wherein each of "Am", "Cm", "Gm" and "Um" are a modified base having a 2'-O-methyl group.

### References

1. Ascano M, Gerstberger S, Tuschl T. Multi-disciplinary methods to define RNA-protein interactions and regulatory networks. Curr Opin Genet Dev 2013; 23: 20-8.
2. Riley KJ, Steitz JA. The "Observer Effect" in genome-wide surveys of protein-RNA interactions. Mol Cell 2013; 49: 601-4.
3. Faoro C, Ataide SF. Ribonomic approaches to study the RNA-binding proteome. FEBS Lett 2014; 588: 3649-64.
4. Ule J, Jensen KB, Ruggiu M, Mele A, Ule A, Darnell RB. CLIP identifies Nova-regulated RNA networks in the brain. Science 2003; 302: 1212-5.
5. Darnell RB. HITS-CLIP: panoramic views of protein-RNA regulation in living cells. Wiley Interdiscip Rev RNA 2010; 1: 266-86.
6. Konig J, Zarnack K, Luscombe NM, Ule J. Protein-RNA interactions: new genomic technologies and perspectives. Nat Rev Genet 2012; 13: 77-83.
7. Bernstein DS, Buter N, Stumpf C, Wickens M. Analyzing mRNA-protein complexes using a yeast three-hybrid system. Methods 2002; 26: 123-41.
8. Siprashvili Z, Webster DE, Kretz M, et al. Identification of proteins binding coding and non-coding human RNAs using protein microarrays. BMC Genomics 2012; 13: 633.
9. Butter F, Scheibe M, Morl M, Mann M. Unbiased RNA-protein interaction screen by quantitative proteomics. Proc Natl Acad Sci U S A 2009; 106: 10626-31.
10. Baltz AG, Munschauer M, Schwanhausser B, et al. The mRNA-bound proteome and its global occupancy profile on protein-coding transcripts. Mol Cell 2012; 46: 674-90.
11. Castello A, Fischer B, Eichelbaum K, et al. Insights into RNA biology from an atlas of mammalian mRNA-binding proteins. Cell 2012; 149: 1393-406.
12. Mitchell SF, Jain S, She M, Parker R. Global analysis of yeast mRNPs. Nat Struct Mol Biol 2013; 20: 127-33.
13. Kwon SC, Yi H, Eichelbaum K, et al. The RNA-binding protein repertoire of embryonic stem cells. Nat Struct Mol Biol 2013; 20: 1122-30.
14. Beckmann BM, Horos R, Fischer B, et al. The RNA-binding proteomes from yeast to man harbour conserved enigmRBPs. Nat Commun 2015; 6: 10127.
15. Conrad T, Albrecht AS, de Melo Costa VR, Sauer S, Meierhofer D, Orom UA. Serial interactome capture of the human cell nucleus. Nat Commun 2016; 7: 11212.
16. Liepelt A, Naarmann-de Vries IS, Simons N, et al. Identification of RNA-binding Proteins in Macrophages by Interactome Capture. Mol Cell Proteomics 2016; 15: 2699-714.
17. Cook KB, Kazan H, Zuberi K, Morris Q, Hughes TR. RBPDB: a database of RNA-binding specificities. Nucleic Acids Res 2010; 39: D301-8.
18. Pashev IG, Dimitrov SI, Angelov D. Crosslinking proteins to nucleic acids by ultraviolet laser irradiation. Trends Biochem Sci 1991; 16: 323-6.
19. Forster AC, Symons RH. Self-cleavage of plus and minus RNAs of a virusoid and a structural model for the active sites. Cell. 1987 Apr 24;49(2):211-20.
20. Pley HW1, Flaherty KM, McKay DB. Three-dimensional structure of a hammerhead ribozyme. Nature. 1994 Nov 3;372(6501):68-74.

## Claims

1. A method for isolating cellular RNA Binding Proteins comprising:
a. UV cross-linking a sample of cells;
b. preparing a lysate of the cells obtained from step (a);
c. digesting the cell lysate obtained from step (b) with DNase;
d. annealing one or more target RNA molecules in the cell lysate from step (c) to RNA splint molecules, and splint ligating the annealed target RNA molecules to RNA adapter molecules to form target RNA: RNA adapter molecules; wherein the RNA adapter molecules are annealed to the RNA splint molecules before, simultaneously, or after annealing of the one or more target RNA molecules to the RNA split molecules;
e. immobilizing the target RNA: RNA adapter molecules of step (d) on a solid phase;
f. washing the immobilized target RNA:RNA adapter molecules of step (e) under denaturing conditions; and either
g. digesting the washed immobilized target RNA: RNA adapter molecules of step (f) with non-specific RNase to release RNA Binding Proteins;
or,
h. providing antisense DNA-based oligonucleotides comprising a nucleotide sequence complementary to an RNA sequence of a selected species of target RNA molecule in the washed immobilized target RNA:RNA adapter molecules of step (f); and annealing said oligonucleotides to said target RNA: RNA adapter molecules to form complementary DNA:RNA heteroduplexes, and then digesting the DNA:RNA heteroduplexes by adding RNaseH to release RNA Binding Proteins bound to the selected species of target RNA molecule,
or,
i. providing a trans-acting sequence-specific ribozyme comprising 5' and 3' antisense oligonucleotides, where the antisense oligonucleotides anneal to the ribonucleotides abutting the cleavage site of the selected species of target RNA molecule, to release RNA Binding Proteins bound to the selected species of target RNA molecule.

2. The method of claim 1, wherein the target RNA molecules are polyA+ RNA molecules.

3. The method of claim 1 or 2, further comprising the step of isolating and identifying the RNA Binding Proteins released in either of step g), h) or i).

4. The method of any one of claims 1-3, wherein the RNA adapter molecules comprise, or are covalently-linked to an affinity group capable of immobilization on the solid phase.

5. The method of claim 4, wherein the solid phase comprises a capture molecule, and wherein said capture molecule is capable of binding to the affinity group and mediating immobilization of the target RNA: RNA adapter molecules.

6. The method of any one of claims 1-5, wherein the cell is a mammalian cell selected from the group consisting of human, mouse and rat cell.

7. The method of any one of claims 1-6, wherein a T4 RNA ligase 2 is used for splint ligating in step (d).

8. The method of any one of claims 1-7, wherein the RNA splint molecule comprises a nucleotide sequence complementary to a 3' end sequence of the target RNA molecule.

9. The method of any one of claims 1-7, wherein the RNA splint molecule comprises a nucleotide sequence complementary to a 5' end sequence of the target RNA molecule.

10. The method of any one of claims 1-9, wherein the antisense DNA-based oligonucleotides are 20 to 30 nucleotides in length and comprise a sequence of 7 to 10 deoxynucleotides flanked at 5' and 3' ends by a sequence of 5 to 10 2'-O-methylated ribonucleotides.

11. The method of any one of claims 1 - 10, wherein the RNA adapter molecule comprises an affinity molecule showing affinity for a capture molecule capable of immobilization on beads or on a column.

12. The method of any one of claims 1-11, wherein in step f) the immobilized target RNA: RNA adapter molecules are washed successively with:
a. a high salt wash buffer comprising 50mM Tris-HCl pH 7.4, 1M NaCl, 1mM EDTA, 0.1% SDS, 0.5% Sodium deoxycholate, and 1% nonyl phenoxypolyethoxylethanol (NP-40);
b. a no-salt wash buffer comprising 20mM Tris-HCl Ph7.4, and 0.2% Tween 20; and
c. an RNase-compatible wash buffer comprising 10mM Tris-HCl pH 7.4, 150mM NaCl, 0,05% NP40, and 2mM EDTA.

13. The method of any one of claims 1-12, wherein the one or more target RNA molecules in step d) is a selected target RNA molecule truncated at the 5' or 3' end by site specific cleavage using a hammerhead ribozyme comprising a 5' and a 3' antisense oligonucleotide, wherein the 5' and 3' antisense oligonucleotides anneal to the ribonucleotides in the selected target RNA molecule that abut the cleavage site.

14. The method of any one of claims 1-13, wherein the DNase in c) is an RNase-free DNase 1 (EC. 3.1.21.1).
